# EUROPEAN PATENT SPECIFICATION

(11) **EP 0 942 919 B1**
(45) Date of publication and mention of the grant of the patent: **21.05.2003**
(21) Application number: 97947778.3
(22) Date of filing: 04.12.1997
(51) Int. Cl.: C07J 41/00

(54) **Sulphatase Inhibitors**
Sulfataseinhibitoren
Inhibiteurs de Sulphatase

(30) Priority: 05.12.1996 GB 9625334
(43) Date of publication of application: 22.09.1999
(60) Divisional application: 02080557.8
(73) Proprietor: Sterix Limited, Oxford OX4 4GA (GB)
(72) Inventor: REED, Michael J., Imperial College School Medicine, Paddington, Loindon W2 1PG (GB); POTTER, Barry V.L., University of Bath, Bath BA2 7AY (GB)
(74) Representative: Harding, Charles Thomas
(86) International application number: GB9703352
(87) International publication number: WO98024802

(56) References cited:
- WO-A-93/05064
- DE-A- 4 429 398
- US-A- 4 668 668
- L. WOO ET AL: "Active Site Directed Inhibition of Estrone Sulfatase by Nonsteroidal Coumarin Sulfamates" JOURNAL OF MEDICINAL CHEMISTRY., vol. 39, no. 7, 29 March 1996, WASHINGTON US, pages 1349-1351, XP002059345
- J. FISHMAN ET AL: "Studies on the Directive O-Methylation of Catechol Estrogens" JOURNAL OF THE AMERICAN CHEMICAL SOCIETY., vol. 89, no. 26, 1967, DC US, pages 7147-7148, XP002059346
- R. PETERS ET AL: "Analogues of [(Triethylsily)ethynyl]estradiol as Potential Antifertility Agents" JOURNAL OF MEDICINAL CHEMISTRY., vol. 31, no. 3, 1988, WASHINGTON US, pages 572-576, XP002059347
- R. RAJAN ET AL: "Estrogen Effects on NADH Oxidase and Superoxide Dismutase in Prepubertal Female Rats" STEROIDS., vol. 40, no. 6, 1982, SAN FRANCISCO US, pages 651-660, XP002059348
- M. CUSHMAN ET AL: "Synthesis, Antitubulin and Antimitotic Activity, and Cytotoxicity of Analogs of 2-Methoxyestradiol, an Endogenous Mammalian Metabolite of Estradiol That Inhibits Tubulin Polymerization by Binding to the Colchicine Binding Site" JOURNAL OF MEDICINAL CHEMISTRY., vol. 38, no. 12, 9 June 1995, WASHINGTON US, pages 2041-2049, XP002059349
- C. LOVELY ET AL: "2-(Hydroxyalkyl)estradiols: Synthesis and Biological Evaluation" JOURNAL OF MEDICINAL CHEMISTRY., vol. 39, no. 9, 26 April 1996, WASHINGTON US, pages 1917-1923, XP002059350
- CHEMICAL ABSTRACTS, vol. 107, no. 21, 23 November 1987 Columbus, Ohio, US; abstract no. 190359, S. BROOKS ET AL: "A-Ring substituted estrogens as inhibitors of the MXT transplantable mammary ductal carcinoma" page 18; column 2; XP002059352 & CANCER RESEARCH, vol. 47, no. 17, 1987, pages 4623-4629,
- TOWNSLEY J D: "Further studies on the regulation of human placental steroid 3-sulfatase activity" ENDOCRINOLOGY, vol. 93, no. 1, 1973, pages 172-181, XP002068155
- CHEMICAL ABSTRACTS, vol. 068, no. 1, 1 January 1968 Columbus, Ohio, US; abstract no. 009640, ADAMS J B: "Enzymic synthesis of steroid sulfates. V. Binding of estrogens to estrogen sulfotransferase" page 913; column 2; XP002068156 & BIOCHIMICA ET BIOPHYSICA ACTA , vol. 146, no. 2, 1967, pages 522-528,
- S. SCHWARZ ET AL: "Synthesis of estrogen sulfamates: Compounds with a novel endocrinological profile" STEROIDS., vol. 61, no. 12, December 1996, SAN FRANCISCO US, pages 710-717, XP002059351

## Description

Evidence suggests that oestrogens are the major mitogens involved in promoting the growth of tumours in endocrine-dependent tissues, such as the breast and endometrium. Although plasma oestrogen concentrations are similar in women with or without breast cancer, breast tumour oestrone and oestradiol levels are significantly higher than in normal breast tissue or blood. *In situ* synthesis of oestrogen is thought to make an important contribution to the high levels of oestrogens in tumours and therefore specific inhibitors of oestrogen biosynthesis are of potential value for the treatment of endocrine-dependent tumours.

Over the past two decades, there has been considerable interest in the development of inhibitors of the aromatase pathway which converts the androgen precursor androstenedione to oestrone. However, there is now evidence that the oestrone sulphatase (E1-STS) pathway, i.e. the hydrolysis of oestrone sulphate to oestrone (E1S to E1), as opposed to the aromatase pathway, is the major source of oestrogen in breast tumours^{1,2}. This theory is supported by a modest reduction of plasma oestrogen concentration in postmenopausal women with breast cancer treated by aromatase inhibitors, such as aminoglutethimide and 4-hydroxyandrostenedione^{3,4} and also by the fact that plasma E1S concentration in these aromatase inhibitor-treated patients remains relatively high. The long half-life of E1S in blood (10-12 h) compared with the unconjugated oestrogens (20 min)⁵ and high levels of steroid sulphatase activity in liver and, normal and malignant breast tissues, also lend support to this theory⁶.

PCT/GB92/01587 teaches novel steroid sulphatase inhibitors and pharmaceutical compositions containing them for use in the treatment of oestrone dependent tumours, especially breast cancer. These steroid sulphatase inhibitors are sulphamate esters. such as N,N-dimethyl oestrone-3-sulphamate and, preferably, oestrone-3-sulphamate (otherwise known as "EMATE").

Some of the compounds disclosed in PCT/GB92/01587 are shown in Figure 1.

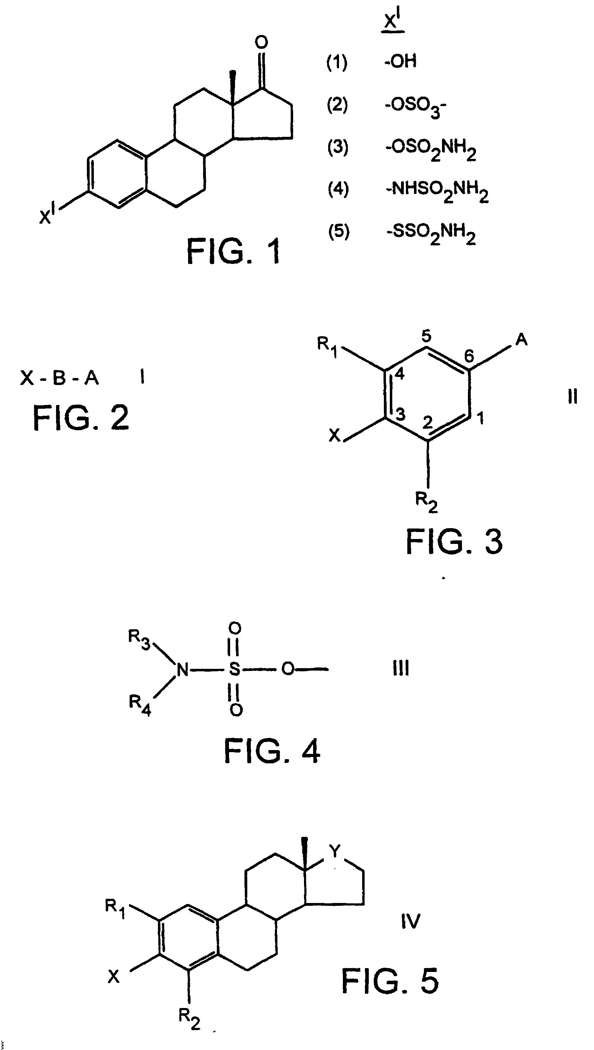

It is known that EMATE is a potent E1-STS inhibitor as it displays more than 99% inhibition of E1-STS activity in intact MCF-7 cells at 0.1 *m*M. EMATE also inhibits the E1-STS enzyme in a time- and concentration-dependent manner, indicating that it acts as an active site-directed inactivator^{7,8}. Although EMATE was originally designed for the inhibition of E1-STS, it also inhibits dehydroepiandrosterone sulphatase (DHA-STS), which is an enzyme that is believed to have a pivotal role in regulating the biosynthesis of the oestrogenic steroid androstenediol^{8,9}. Also, there is now evidence to suggest that androstenediol may be of even greater importance as a promoter of breast tumour growth¹⁰. EMATE is also active *in vivo* as almost complete inhibition of rat liver E1-STS (99%) and DHA-STS (99%) activities resulted when it is administered either orally or subcutaneously¹¹. In addition, EMATE has been shown to have a memory enhancing effect in rats¹⁴. Studies in mice have suggested an association between DHA-STS activity and the regulation of part of the immune response. It is thought that this may also occur in humans^{15,16}. The bridging *O*-atom of the sulphamate moiety in EMATE is important for inhibitory activity. Thus, when the 3-*O*-atom is replaced by other heteroatoms (Figure 1) as in oestrone-3-*N*-sulphamate (4) and oestrone-3-*S*-sulphamate (5), these analogues are weaker non-time-dependent inactivators¹².

Although optimal potency for inhibition of E1-STS may have been attained in EMATE, it is possible that oestrone may be released during sulphatase inhibition^{8,12}, and that EMATE and its oestradiol congener may possess oestrogenic activity¹³.

The present invention seeks to provide novel compounds suitable for the inhibition of E1-STS but preferably wherein those compounds have no, or a minimal, oestrogenic effect.

According to a first aspect of the present invention there is provided a sulphamate compound suitable for use as an inhibitor of oestrone sulphatase, wherein the compound is a sulphamate compound having Formula V; 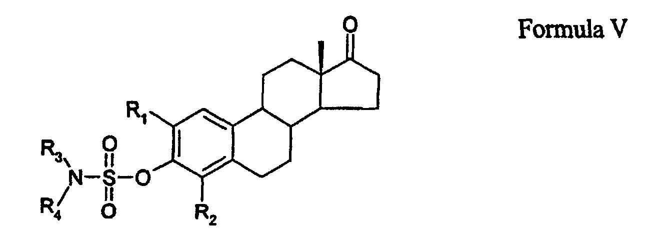 wherein each of R₁ and R₂ is independently selected from H, alkyl, cycloalkyl, alkoxy, alkenyl, aryl, substituted alkyl, substituted cycloalkyl, substituted alkenyl, substituted aryl, a nitrogen containing group, a S containing group, or a carboxy containing group; R₁ and R₂ may be the same or different but not both being H; and each of R₃ and R₄ is independently selected from H, alkyl, cycloalkyl, alkenyl and aryl, wherein at least one of R₃ and R₄ is H.

According to a second aspect of the present invention there is provided a sulphamate compound suitable for use as an inhibitor of oestrone sulphatase, wherein the compound is a sulphamate compound having Formula II; 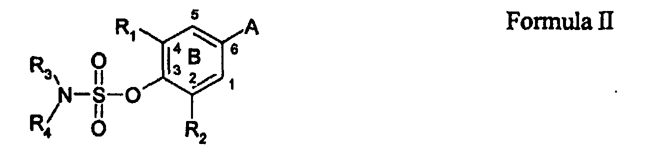 wherein R₁ is selected from alkyl, cycloalkyl, alkoxy, alkenyl, aryl, substituted alkyl, substituted cycloalkyl, substituted alkenyl, substituted aryl, a nitrogen containing group, a S containing group, or a carboxy containing group; R₂ is selected from H, alkyl, cycloalkyl, alkoxy, alkenyl, aryl, substituted alkyl, substituted cycloalkyl, substituted alkenyl, substituted aryl, a nitrogen containing group, a S containing group, or a carboxy containing group; R₁ and R₂ may be the same or different; each of R₃ and R₄ is independently selected from H, alkyl, cycloalkyl, alkenyl and aryl, wherein at least one of R₃ and R₄ is H; group A and ring B together are capable of mimicking the A and B rings of oestrone; and group A is additionally attached to the carbon atom at position 1 of the ring B.

According to a third aspect of the present invention there is provided use of a compound in the manufacture of a medicament to inhibit steroid sulphatase activity, wherein the compound is a sulphamate compound having Formula II; 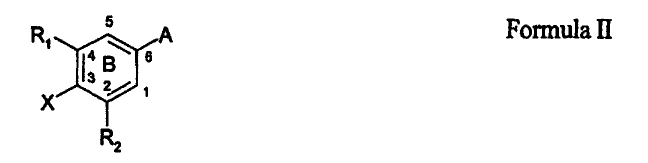 wherein X is a sulphamate group; R₁ is selected from alkyl, cycloalkyl, alkoxy, alkenyl, aryl, substituted alkyl, substituted cycloalkyl, substituted alkenyl, substituted aryl, a nitrogen containing group, a S containing group, or a carboxy containing group; and R₂ is selected from H, alkyl, cycloalkyl, alkoxy, alkenyl, aryl, substituted alkyl, substituted cycloalkyl, substituted alkenyl, substituted aryl, a nitrogen containing group, a S containing group, or a carboxy containing group; R₁ and R₂ may be the same or different; wherein group A and ring B together are capable of mimicking the A and B rings of oestrone; and wherein group A is additionally attached to the carbon atom at position 1 of the ring B.

According to a fourth aspect of the present invention there is provided a 4. Use of a compound in the manufacture of a medicament to inhibit steroid sulphatase activity, wherein the compound is a sulphamate compound having Formula V; 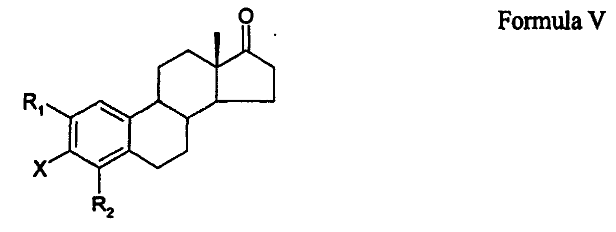 wherein X is a sulphamate group; each of R₁ and R₂ is independently selected from H, alkyl, cycloalkyl, alkoxy, alkenyl, aryl, substituted alkyl, substituted cycloalkyl, substituted alkenyl, substituted aryl, a nitrogen containing group, a S containing group, or a carboxy containing group; and R₁ and R₂ may be the same or different but not both being H.

The term "mimic" as used herein means having a similar or different structure but having a similar functional effect. In otherwords, group A and ring B together of the compounds of the present invention are bio-isosteres of the A and B rings of oestrone.

A key advantage of the present invention is that the sulphamate compounds of the present invention can act as E1-STS inhibitors.

Another advantage of the compounds of the present invention is that they may be potent *in vivo* and that they may have less oestrogenic activity than the known compounds and can therefore be deemed to be a "non-oestrogenic compound". The term "non-oestrogenic compound" as used herein means a compound exhibiting no or substantially no oestrogenic activity.

The present invention therefore provides sulphamate compounds which may have a reduced oestrogenic activity.

Another advantage is that the compounds may not be capable of being metabolised to compounds which display or induce hormonal activity.

The compounds of the present invention are also advantageous in that they may be orally active.

The compounds of the present invention are further advantageous in that they may have an irreversible effect.

In a preferred embodiment, the sulphamate compounds of the present invention are useful for the treatment of breast cancer.

In addition, the sulphamate compounds of the present invention are useful for the treatment of non-malignant conditions, such as the prevention of auto-immune diseases, particularly when pharmaceuticals may need to be administered from an early age.

The sulphamate compounds of the present invention are also believed to have therapeutic uses other than for the treatment of endocrine-dependent cancers, such as the treatment of autoimmune diseases.

Preferably, group A and ring B are a steroid ring structure or a substituted derivative thereof.

The term "sulphamate" as used herein includes an ester of sulphamic acid, or an ester of an N-substituted derivative of sulphamic acid, or a salt thereof.

Preferably, the sulphamate group has the Formula III.

R₃ and R₄ are independently selected from H or alkyl, cycloalkyl, alkenyl and aryl, or together represent alkylene, wherein the or each alkyl or cycloalkyl or alkenyl or optionally contain one or more hetero atoms or groups.

When substituted, the N-substituted compounds of this invention may contain one or two N-alkyl, N-alkenyl, N-cycloalkyl or N-aryl substituents, preferably containing or each containing a maximum of 10 carbon atoms. When R₃ and/or R₄ is alkyl, the preferred values are those where R₃ and R₄ are each independently selected from lower alkyl groups containing from 1 to 5 carbon atoms, that is to say methyl, ethyl, propyl etc. Preferably R₃ and R₄ are both methyl. When R₃ and/or R₄ is aryl, typical values are phenyl and tolyl (-PhCH₃; *o*-, *m*- or *p*-). Where R₃ and R₄ represent cycloalkyl, typical values are cyclopropyl, cyclopentyl, cyclohexyl etc. When joined together R₃ and R₄ typically represent an alkylene group providing a chain of 4 to 6 carbon atoms, optionally interrupted by one or more hetero atoms or groups, e.g. -0- or -NH- to provide a 5-, 6- or 7- membered heterocycle, e.g. morpholino, pyrrolidino or piperidino.

Within the values alkyl, cycloalkyl, alkenyl and aryl we include substituted groups containing as substituents therein one or more groups which do not interfere with the sulphatase inhibitory activity of the compound in question. Exemplary non-interfering substituents include hydroxy, amino, halo, alkoxy, alkyl and aryl.

In some preferred embodiments, at least one of R₃ and R₄ is H.

In some further preferred embodiments, each of R₃ and R₄ is H.

Preferably, each of R₁ and R₂ is independently selected from H, alkyl, cycloalkyl, alkenyl, aryl, substituted alkyl, substituted cycloalkyl, substituted alkenyl, substituted aryl, any other suitable hydrocarbyl group, a nitrogen containing group, a S containing group, a carboxy containing group.

Likewise, here, the term "hydrocarbyl group" means a group comprising at least C and H and may optionally comprise one or more other suitable substituents. Examples of such substituents may include halo-, alkoxy-, nitro-, an alkyl group, a cyclic group etc. In addition to the possibility of the substituents being a cyclic group, a combination of substituents may form a cyclic group. If the hydrocarbyl group comprises more than one C then those carbons need not necessarily be linked to each other. For example, at least two of the carbons may be linked *via* a suitable element or group. Thus, the hydrocarbyl group may contain hetero atoms. Suitable hetero atoms will be apparent to those skilled in the art and include, for instance, sulphur, nitrogen and oxygen. A non-limiting example of a hydrocarbyl group is an acyl group.

Preferably, each of R₁ and R₂ is independently selected from H, C₁₋₆ alkyl, C₁₋₆ cycloalkyl, C₁₋₆ alkenyl, substituted C₁₋₆ alkyl, substituted C₁₋₆ cycloalkyl, substituted C₁₋₆ alkenyl, substituted aryl, a nitrogen containing group, a S containing group, or a carboxy group having from 1-6 carbon atoms.

Likewise, here within the values alkyl, cycloalkyl, alkenyl and aryl we include substituted groups containing as substituents therein one or more groups which do not interfere with the sulphatase inhibitory activity of the compound in question. Exemplary non-interfering substituents include hydroxy, amino, halo, alkoxy, alkyl and aryl.

Preferably, each of R₁ and R₂ is independently selected from H, C₁₋₆ alkyl, C₁₋₆ alkenyl, a nitrogen containing group, or a carboxy group having from 1-6 carbon atoms.

Preferably, each of R₁ and R₂ is independently selected from H, C₁₋₆ alkyl, C₁₋₆ alkenyl, NO₂, or a carboxy containing group having from 1-6 carbon atoms.

Preferably, each of R₁ and R₂ is independently selected from H, C₃ alkyl, C₃ alkenyl, NO₂, or H₃CO.

Preferably, the compound is any one of the Formulae V - IX.

Preferably, for some applications, the compound is further characterised by the feature that if the sulphamate group were to be substituted by a sulphate group to form a sulphate derivative, then the sulphate derivative would be hydrolysable by an enzyme having steroid sulphatase (E.C. 3.1.6.2) activity - i.e. when incubated with steroid sulphatase EC 3.1.6.2 at pH 7.4 and 37°C.

In one preferred embodiment, if the sulphamate group of the compound were to be replaced with a sulphate group to form a sulphate compound then that sulphate compound would be hydrolysable by an enzyme having steroid sulphatase (E.C. 3.1.6.2) activity and would yield a Kₘ value of less than 50mmolar when incubated with steroid sulphatase EC 3.1.6.2 at pH 7.4 and 37°C.

In another preferred embodiment, if the sulphamate group of the compound were to be replaced with a sulphate group to form a sulphate compound then that sulphate compound would be hydrolysable by an enzyme having steroid sulphatase (E.C. 3.1.6.2) activity and would yield a Kₘ value of less than 50µmolar when incubated with steroid sulphatase EC 3.1.6.2 at pH 7.4 and 37°C.

In a highly preferred embodiment, the compound of the present invention is not hydrolysable by an enzyme having steroid sulphatase (E.C. 3.1.6.2) activity.

Thus, the present invention provides novel sulphamate compounds.

Preferably the group A and the ring B together - hereinafter referred to as "group A/ring B combination" - will contain, inclusive of all substituents, a maximum of about 50 carbon atoms, more usually no more than about 30 to 40 carbon atoms.

A preferred group A/ring B combination has a steroidal ring structure, that is to say a cyclopentanophenanthrene skeleton. Preferably, the sulphamyl or substituted sulphamyl group is attached to that skeleton in the 3-position.

Thus, according to a preferred embodiment, the group A/ring B combination is a substituted or unsubstituted, saturated or unsaturated steroid nucleus.

A suitable steroid nucleus is a substituted (i.e. substituted in at least the 2 and/or 4 position and optionally elsewhere in the steroid nucleus) derivative of any one of: oestrone, 2-OH-oestrone, 2-methoxy-oestrone, 4-OH-oestrone, 6a-OH-oestrone, 7a-OH-oestrone, 16a-OH-oestrone, 16b-OH-oestrone, oestradiol, 2-OH-17b-oestradiol, 2-methoxy-17b-oestradiol, 4-OH-17b-oestradiol, 6a-OH-17b-oestradiol, 7a-OH-17b-oestradiol, 16a-OH-17a-oestradiol, 16b-OH-17a-oestradiol, 16b-OH-17b-oestradiol, 17a-oestradiol, 17b-oestradiol, 17a-ethinyl-17b-oestradiol, oestriol, 2-OH-oestriol, 2-methoxy-oestriol, 4-OH-oestriol, 6a-OH-oestriol, 7a-OH-oestriol, dehydroepiandrosterone, 6a-OH-dehydroepiandrosterone, 7a-OH-dehydroepiandrosterone, 16a-OH-dehydroepiandrosterone, 16b-OH-dehydroepiandrosterone.

In general terms the group A/ring B combination may contain a variety of non-interfering substituents. In particular, the group A/ring B combination may contain one or more hydroxy, alkyl especially lower (C₁-C₆) alkyl, e.g. methyl, ethyl, n-propyl, isopropyl, n-butyl, sec-butyl, tert-butyl, n-pentyl and other pentyl isomers, and n-hexyl and other hexyl isomers, alkoxy especially lower (C₁-C₆) alkoxy, e.g. methoxy, ethoxy, propoxy etc., alkenyl, e.g. ethenyl, or halogen, e.g. fluoro substituents.

The group A/ring B combination may even be a non-steroidal ring system.

A suitable non-steroidal ring system is a substituted (i.e. substituted in at least the 2 and/or 4 position and optionally elsewhere in the ring system) derivative of any one of: diethylstilboestrol, stilboestrol.

When substituted, the N-substituted compounds of this invention may contain one or two N-alkyl, N-alkenyl, N-cycloalkyl or N-aryl substituents, preferably containing or each containing a maximum of 10 carbon atoms.

When R₁ and/or R₂ and/or R₃ and/or R₄ is alkyl, the preferred values are those where each of R₁ and R₂ and R₃ and R₄ is independently selected from lower alkyl groups containing from 1 to 6 carbon atoms, that is to say methyl, ethyl or propyl.

When R₁ and/or R₂ and/or R₃ and/or R₄ is aryl, typical groups are phenyl and tolyl (-PhCH₃; *o-*, *m-* or *p*-).

Where R₁ and/or R₂ and/or R₃ and/or R₄ represent cycloalkyl, typical values are cyclopropyl, cyclopentyl or cyclohexyl.

When joined together R₃ and R₄ typically represent an alkylene group providing a chain of 4 to 6 carbon atoms, optionally interrupted by one or more hetero atoms or groups, e.g. -0- or -NH- to provide a 5-, 6- or 7- membered heterocycle, e.g. morpholino, pyrrolidino or piperidino.

Within the values alkyl, cycloalkyl, alkenyl and aryl we include substituted groups containing as substituents therein one or more groups which do not interfere with the sulphatase inhibitory activity of the compound in question. Examples of non-interfering substituents include hydroxy, amino, halo, alkoxy, alkyl and aryl.

Any replacement for H on the ring system may be any one of the substituents described above in relation to R₁ and R₂.

According to a further aspect of the present invention there is provided a sulphamate compound according to the present invention for use as a pharmaceutical.

According to a further aspect of the present invention there is provided a sulphamate compound according to the present invention for inhibiting oestrone sulphatase.

According to a further aspect of the present invention there is provided a pharmaceutical composition comprising a sulphamate compound according to the present invention; and a pharmaceutically acceptable carrier, excipient, adjuvant or diluent.

According to a further aspect of the present invention there is provided the use of a sulphamate compound according to the present invention in the manufacture of a pharmaceutical for inhibiting oestrone sulphatase.

The sulphamate compounds of the present invention may be prepared by reacting an appropriate alcohol with a sulfamoyl chloride, R₃R₄NSO₂Cl.

Preferred conditions for carrying out the reaction are as follows.

Sodium hydride and a sulfamoyl chloride are added to a stirred solution of the alcohol in anhydrous dimethyl formamide at 0°C. Subsequently, the reaction is allowed to warm to room temperature whereupon stirring is continued for a further 24 hours. The reaction mixture is poured onto a cold saturated solution of sodium bicarbonate and the resulting aqueous phase is extracted with dichloromethane. The combined organic extracts are dried over anhydrous MgSO₄. Filtration followed by solvent evaporation *in vacuo* and co-evaporated with toluene affords a crude residue which is further purified by flash chromatography.

Preferably, the alcohol is derivatised, as appropriate, prior to reaction with the sulfamoyl chloride. Where necessary, functional groups in the alcohol may be protected in known manner and the protecting group or groups removed at the end of the reaction.

For pharmaceutical administration, the steroid sulphatase inhibitors of this invention can be formulated in any suitable manner utilising conventional pharmaceutical formulating techniques and pharmaceutical carriers, adjuvants, excipients, diluents etc. and usually for parenteral administration. Approximate effective dose rates are in the range 100 to 800 mg/day depending on the individual activities of the compounds in question and for a patient of average (70Kg) bodyweight. More usual dosage rates for the preferred and more active compounds will be in the range 200 to 800 mg/day, more preferably, 200 to 500 mg/day, most preferably from 200 to 250 mg/day. They may be given in single dose regimes, split dose regimes and/or in multiple dose regimes lasting over several days. For oral administration they may be formulated in tablets, capsules, solution or suspension containing from 100 to 500 mg of compound per unit dose. Alternatively and preferably the compounds will be formulated for parenteral administration in a suitable parenterally administrable carrier and providing single daily dosage rates in the range 200 to 800 mg, preferably 200 to 500, more preferably 200 to 250 mg. Such effective daily doses will, however, vary depending on inherent activity of the active ingredient and on the bodyweight of the patient, such variations being within the skill and judgement of the physician.

For particular applications, it is envisaged that the steroid sulphatase inhibitors of this invention may be used in combination therapies, either with another sulphatase inhibitor, or, for example, in combination with an aromatase inhibitor, such as for example, 4-hydroxyandrostenedione (4-OHA).

In summation, the present invention provides novel compounds for use as steroid sulphatase inhibitors, and pharmaceutical compositions containing them.

The present invention will now be described only by way of example with reference to the accompanying drawings in which:-
Figure 1 shows the known structures of oestrone (1), oestrone sulphate (2), EMATE (3) and steroid sulphamates (4-5);
Figure 2 shows a compound of the Formula I;
Figure 3 shows a compound of the Formula II;
Figure 4 shows a compound of the Formula III;
Figure 5 shows a compound of the Formula IV;

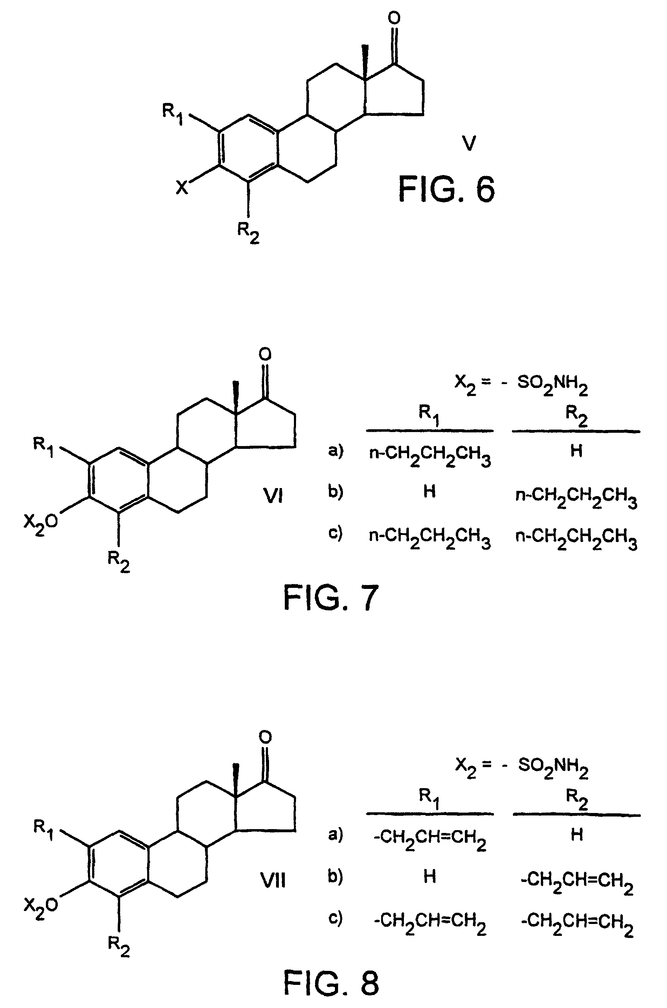

Figure 6 shows a compound of the Formula V;
Figure 7 shows a compound of the Formula VI;
Figure 8 shows a compound of the Formula VII;

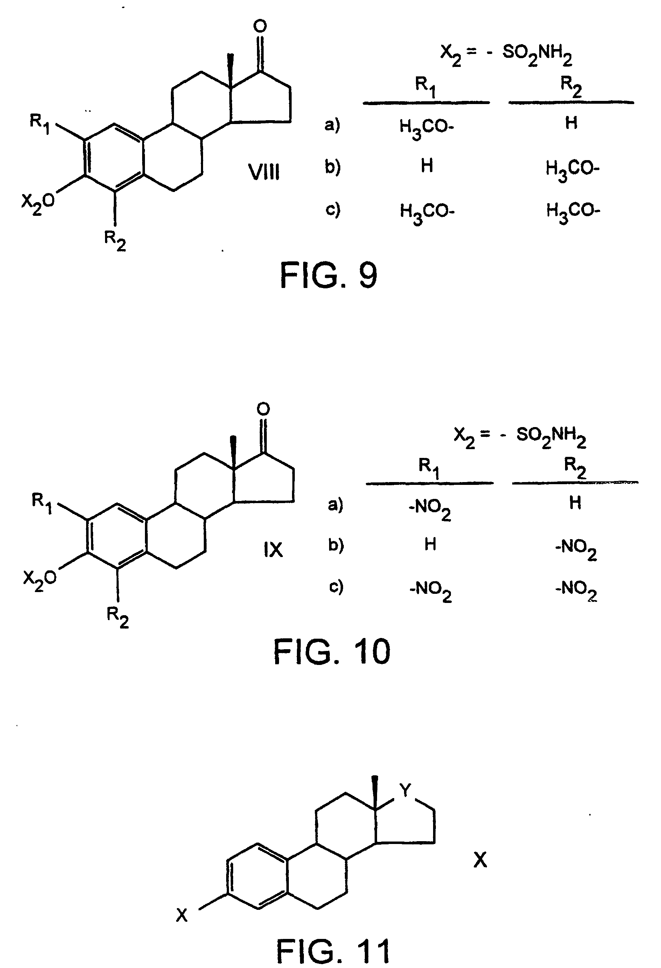

Figure 9 shows a compound of the Formula VIII;
Figure 10 shows a compound of the Formula IX;
Figure 11 shows a compound of the Formula X;
Figure 12 shows one embodiment of a method of preferring compounds of the present invention;
Figure 13 shows another embodiment of a method of preferring compounds of the present invention;
Figure 14 shows yet another embodiment of a method of preferring compounds of the present invention;
Figure 15 shows a further embodiment of a method of preferring compounds of the present invention;
Figure 16 shows a graph illustrating the *in vivo* inhibition of oestrone sulphatase by NOMATE (0.1 mg/Kg/day for five days); and
Figure 17 shows a graph illustrating the lack of effect of NOMATE (0.1 mg/Kg/day for five days) on uterine weights in ovariectomised rats.

The invention will now be described only by way of Examples.

### Example 1- Preparative Methods

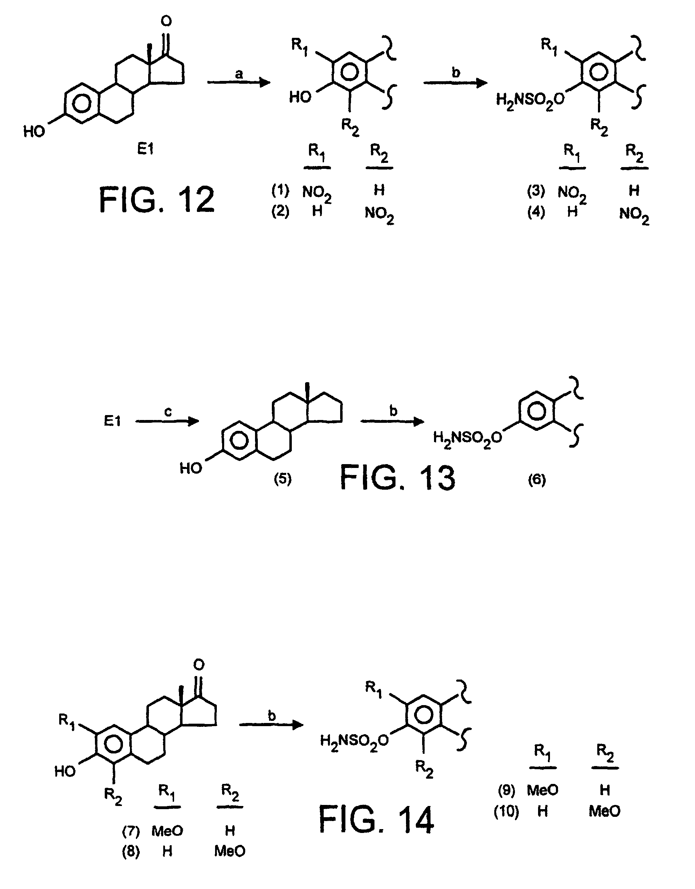

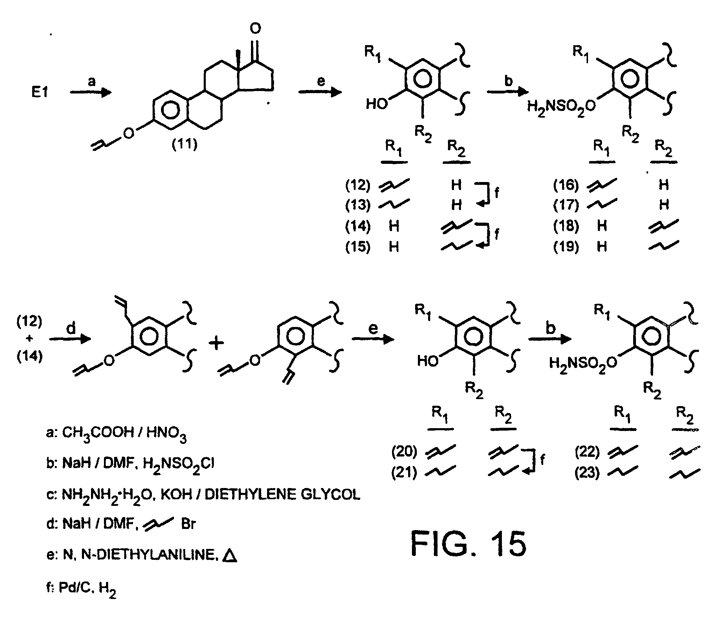

The preparation of various compounds in accordance with the present invention is illustrated in Figures 12 to 15. In these Figures, the curved lines attached to the phenyl rings represent the remainder of the ringed structure.

### Example 1 - In Vitro Inhibition

The ability of compounds to inhibit oestrone sulphatase activity was assessed using either intact MCF-7 breast cancer cells or placental microsomes as previously described¹¹.

In this regard, the teachings of that earlier reference¹¹ are as follows:

### Inhibition of Steroid Sulphatase Activity in MCF-7 cells by oestrone-3-sulphamate

Steroid sulphatase is defined as: Steryl Sulphatase EC 3.1.6.2.

Steroid sulphatase activity was measured *in vitro* using intact MCF-7 human breast cancer cells. This hormone dependent cell line is widely used to study the control of human breast cancer cell growth. It possesses significant steroid sulphatase activity (Maclndoe et al. *Endocrinology,* **123,** 1281-1287 (1988); Purohit & Reed, *Int*. *J. Cancer,* **50**, 901-905 (1992)) and is available in the U.S.A. from the American Type Culture Collection (ATCC) and in the U.K. (e.g. from The Imperial Cancer Research Fund). Cells were maintained in Minimal Essential Medium (MEM) (Flow Laboratories, Irvine, Scotland) containing 20 mM HEPES, 5% foetal bovine serum, 2 mM glutamine, non-essential amino acids and 0.075% sodium bicarbonate. Up to 30 replicate 25 cm² tissue culture flasks were seeded with approximately 1 x 10⁵ cells/flask using the above medium. Cells were grown to 80% confluency and medium was changed every third day.

Intact monolayers of MCF-7 cells in triplicate 25 cm² tissue culture flasks were washed with Earle's Balanced Salt Solution (EBSS from ICN Flow, High Wycombe. U.K.) and incubated for 3-4 hours at 37°C with 5 pmol (7 x 10⁵ dpm) [6,7-³H]oestrone-3-sulphate (specific activity 60 Ci/mmol from New England Nuclear, Boston, Mass., U.S.A.) in serum-free MEM (2.5 ml) together with oestrone-3-sulphamate (11 concentrations: 0; 1fM; 0.01pM; 0.1pM; 1pM; 0.01nM; 0.1nM; 1nM; 0.01mM; 0.1mM; 1mM). After incubation each flask was cooled and the medium (1 ml) was pipetted into separate tubes containing [¹⁴C]oestrone (7 x 10³ dpm) (specific activity 97 Ci/mmol from Amersham International Radiochemical Centre, Amersham, U.K.). The mixture was shaken thoroughly for 30 seconds with toluene (5 ml). Experiments showed that >90% [¹⁴C]oestrone and <0.1% [³H]oestrone-3-sulphate was removed from the aqueous phase by this treatment. A portion (2 ml) of the organic phase was removed, evaporated and the ³H and ¹⁴C content of the residue determined by scintillation spectrometry. The mass of oestrone-3-sulphate hydrolysed was calculated from the ³H counts obtained (corrected for the volumes of the medium and organic phase used, and for recovery of [¹⁴C]oestrone added) and the specific activity of the substrate. Each batch of experiments included incubations of microsomes prepared from a sulphatase-positive human placenta (positive control) and flasks without cells (to assess apparent non-enzymatic hydrolysis of the substrate). The number of cell nuclei per flask was determined using a Coulter Counter after treating the cell monolayers with Zaponin. One flask in each batch was used to assess cell membrane status and viability using the Trypan Blue exclusion method (Phillips, H.J. (1973) In: *Tissue culture and applications,* [eds: Kruse, D.F. & Patterson, M.K.]; pp. 406-408; Academic Press, New York).

Results for steroid sulphatase activity are expressed as the mean ± 1 S.D. of the total product (oestrone + oestradiol) formed during the incubation period (20 hours) calculated for 10⁶ cells and, for values showing statistical significance, as a percentage reduction (inhibition) over incubations containing no oestrone-3-sulphamate. Unpaired Student's t-test was used to test the statistical significance of results.

### Inhibition of Steroid Sulphatase Activity in Placental Microsomes by Oestrone-3-sulphamate

Sulphatase-positive human placenta from normal term pregnancies (Obstetric Ward, St. Mary's Hospital, London) were thoroughly minced with scissors and washed once with cold phosphate buffer (pH 7.4, 50 mM) then re-suspended in cold phosphate buffer (5 ml/g tissue). Homogenisation was accomplished with an Ultra-Turrax homogeniser, using three 10 second bursts separated by 2 minute cooling periods in ice. Nuclei and cell debris were removed by centrifuging (4°C) at 2000g for 30 minutes and portions (2 ml) of the supernatant were stored at -20°C. The protein concentration of the supernatants was determined by the method of Bradford (*Anal. Biochem*., **72**, 248-254 (1976)).

Incubations (1 ml) were carried out using a protein concentration of 100 mg/ml, substrate concentration of 20 mM [6,7-³H]oestrone-3-sulphate (specific activity 60 Ci/mmol from New England Nuclear, Boston, Mass., U.S.A.) and an incubation time of 20 minutes at 37°C. If necessary eight concentrations of compounds are employed: 0 (i.e. control); 0.05mM; 0.1mM; 0.2mM; 0.4mM; 0.6mM; 0.8mM; 1.0mM. After incubation each sample was cooled and the medium (1 ml) was pipetted into separate tubes containing [¹⁴C]oestrone (7 x 10³ dpm) (specific activity 97 Ci/mmol from Amersham International Radiochemical Centre, Amersham, U.K.). The mixture was shaken thoroughly for 30 seconds with toluene (5 ml). Experiments showed that >90% [¹⁴C]oestrone and <0.1% [³H]oestrone-3-sulphate was removed from the aqueous phase by this treatment. A portion (2 ml) of the organic phase was removed, evaporated and the ³H and ¹⁴C content of the residue determined by scintillation spectrometry. The mass of oestrone-3-sulphate hydrolysed was calculated from the ³H counts obtained (corrected for the volumes of the medium and organic phase used, and for recovery of [¹⁴C]oestrone added) and the specific activity of the substrate.

For the present invention, the percentage inhibition for the series of EMATE analogues tested in either MCF-7 cells or placental microsomes is shown in Table 1.

### Example 2-In Vivo Studies

Using 17-deoxy oestrone-3-O-sulphamate (NOMATE, Figure 5, Formula IV where X = -OSO₂NH₂, Y = -CH₂- and R₁ and R₂ = H, and Figure 13) as a representative example, the ability of this compound to inhibit oestrone sulphatase activity *in vivo* was examined in rats. The oestrogenicity of this compound was examined in ovariectomised rats. In this model compounds which are oestrogenic stimulate uterine growth.

### (i) Inhibition of oestrone sulphatase activity in vivo

NOMATE (0.1 mg/Kg/day for five days) was administered orally to rats with another group of animals receiving vehicle only (propylene glycol). At the end of the study samples of liver tissue were obtained and oestrone sulphatase activity assayed using ³H oestrone sulphate as the substrate as previously described¹¹.

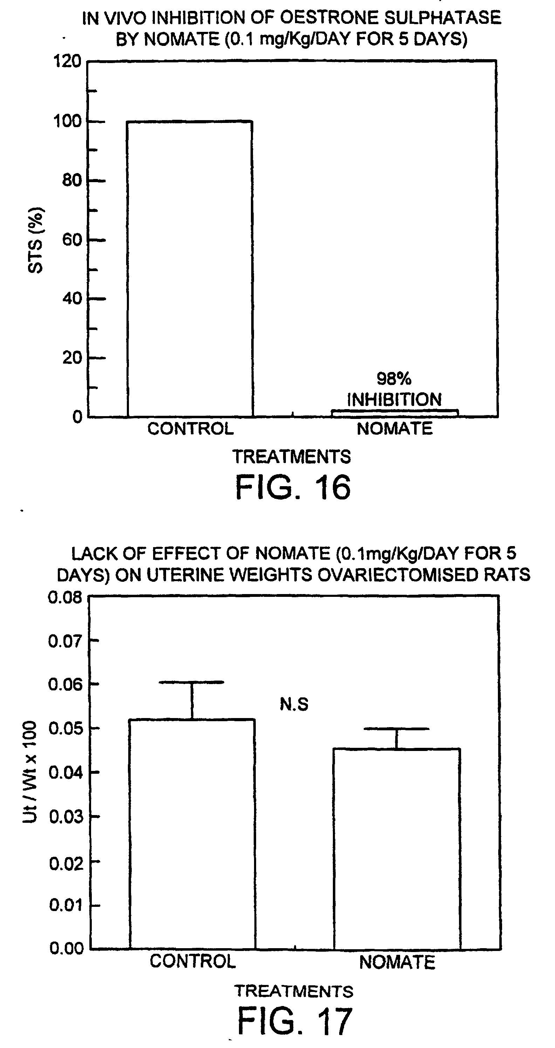

As shown in Figure 16, administration of this dose of NOMATE effectively inhibited oestrone sulphatase activity by 98% compared with untreated controls.

### (ii) Lack of in vivo oestrogenicity

NOMATE (0.1 mg/Kg/day for five days) was administered orally to rats with another group of animals receiving vehicle only (propylene glycol). At the end of the study uteri were obtained and weighed with the results being expressed as uterine weight/whole body weight x 100.

As shown in Figure 17, administration of NOMATE at the dose tested, but had no significant effect on uterine growth, showing that at this dose the compound is not oestrogenic.

**TABLE 1**

| **Inhibition of Oestrone Sulphatase Activity in MCF-7 Cells or Placental Microsomes by EMATE Analogues** | | | |
|---|---|---|---|
| **Inhibitor** | **Concentration Tested (mM)** | **% Inhibition (Mean)** | |
| | | **MCF-7 Cells** | **Placental Microsomes** |
| 2-n-propyl EMATE | 0.1 | 41.1 | - |
| | 1 | 83.1 | 21.9 |
| | 10 | 92.2 | 43.2 |
| | 25 | - | 47.5 |
| | 50 | - | 61.1 |
| | 100 | - | 69.2 |
| | | | |
| 4-n-propyl EMATE | 1 | - | 13.7 |
| | 10 | - | 10.2 |
| | 25 | - | 15.7 |
| | 50 | - | 16.3 |
| | 100 | - | 23.7 |
| | | | |
| 2,4-n-dipropyl EMATE | 0.1 | 6.6 | - |
| | 1 | 10.6 | - |
| | | | |
| 2-allyl EMATE | 0.01 | 23.2 | - |
| | 0.1 | 76.1 | - |
| | 1 | 94.2 | 45.6 |
| | 10 | 93.7 | 65.4 |
| | 25 | - | 75.3 |
| | 50 | - | 86.6 |
| | 100 | - | 89.6 |
| | | | |
| 4-allyl EMATE (approx 75%) | 1 | - | 29.1 |
| | 10 | - | 54.2 |
| | 25 | - | 59.0 |
| | 50 | - | 65.1 |
| | 100 | - | 71.9 |
| | | | |
| 2,4-di-allyl EMATE | - | - | - |
| | | | |
| 2-methoxy EMATE | 0.1 | 96.0 | - |
| | 1 | 93.6 | - |
| | 10 | 96.2 | 99.0 |
| | 50 | - | 99.7 |
| | 100 | - | 99.7 |
| | | | |
| 2-nitro EMATE | 0.05 | - | 44.5 |
| | 0.5 | - | 93.9 |
| | 5 | - | 99.0 |
| | 50 | - | 99.4 |
| | | | |
| 4-nitro EMATE | 20 | - | 99.0 |
| | | | |
| NOMATE (17-deoxy EMATE) | 0.1 | 96.4 | 97.2 |
| | 1 | 99.1 | 99.5 |
| | 10 | 99.7 | 99.5 |
| | 25 | 99.7 | 99.7 |
| - - = not tested | | | |
| - Irreversible time- and concentration-dependent inhibition is assumed for these compounds in keeping with established precedent⁸. | | | |

Other modifications of the present invention will be apparent to those skilled in the art.

### REFERENCES

(1) Santner, S. J.; Feil, P. D.; Santen, R. J. *In situ* oestrogen production via the oestrone sulphatase pathway in breast tumors: relative importance vs. the aromatase pathway. *J. Clin. Endocrinol. Metab.* **1984,** *59*, 29-33.
(2) Yamamoto, T.; Kitawaki, J.; Urabe, M.; Honjo, H.; Tamura, T.; Noguchi, T.; Okada, H.; Sasaki, H.; Tada, A.; Terashima, Y.; Nakamura, J.; Yoshihama, M. Oestrogen productivity of endometrium and endometrial cancer tissue - influence of aromatase on proliferation of endometrial cancer cells. *J. Steroid Biochem. Mol. Biol.* **1993,** *44*, 463-468.
(3) Santen, R. J.; Santner, S. J.; Davis, B.; Veldhuis, J.; Samojilik. E.; Ruby, E. Aminogluthethimide inhibits extraglandular oestrogen production in post-menopausal women with breast carcinoma. *J. Clin. Endocrinol. Metab.* **1978,** *47*, 1257-1265.
(4) Reed, M. J.; Lai, L. C.; Owen, A. M.; Singh, A.; Coldham, N. G.; Purohit, A.; Ghilchik, M. W.; Shaikh, N. A.; James, V. H. T. Effect of treatment with 4-hydroxyandrostenedione on the peripheral conversion of androstenedione to oestrone and *in vitro* tumour aromatase activity in postmenopausal women with breast cancer. *Cancer Res.* **1990,** *50*, 193-196.
(5) Ruder, H. J.; Loriaux, D. L.; Lipsett, M. B. Oestrone sulphate: production rate and metabolism in man. *J. Clin. Invest.* **1972,** *51*, 1020-1023.
(6) James, V. H. T.; McNeill, J. M.; Lai, L. C.; Newton, C. J.; Ghilchik, M. W.; Reed, M. J. Aromatase activity in normal breast and breast tumor tissues: *in vivo* and *in vitro* studies. *Steroids* **1987,** *50*, 269-279.
(7) Howarth, N. M.; Purohit, A.; Reed, M. J.; Potter, B. V. L. Oestrone sulphamates: potent inhibitors of oestrone sulphatase with therapeutic potential. *J. Med. Chem.* **1994,** *37*, 219-221.
(8) Purohit, A.; Williams, G. J.; Howarth, N. M.; Potter, B. V. L.; Reed, M. J. Inactivation of steroid sulphatase by an active site-directed inhibitor, oestrone-3-*O*-sulphamate. *Biochemistry* **1995,** *34*, 11508-11514.
(9) Purohit, A.; Dauvois, S.; Parker, M. G.; Potter, B. V. L.; Williams. G. J.; Reed, M. J. The hydrolysis of oestrone sulphate and dehydroepiandrosterone sulphate by human steroid sulphatase expressed in transfected COS-1 cells. *J. Steroid Biochem. Mol. Biol.* **1994,** *50*, 101-104.
(10) Dauvois, S.; Labrie, F. Androstenedione and androst-5-ene-3*b*,17*b*-diol stimulate DMBA-induced rat mammary tumours - role of aromatase. *Breast Cancer Res. Treat.* **1989,** *13*, 61-69.
(11) Purohit, A.; Williams, G. J.; Roberts, C. J.; Potter, B. V. L.; Reed, M. J. *In vivo* inhibition of oestrone sulphatase and dehydroepiandrosterone sulphatase by oestrone-3-*O*-sulphamate. *Int. J. Cancer* **1995,** *62*, 106-111.
(12) Woo, L. W. L.; Lightowler, M.; Purohit, A.; Reed, M. J.; Potter, B. V. L. Heteroatom-substituted analogues of the active-site directed inhibitor oestra-1,3,5(10)-trien-17-one-3-sulphamate inhibit oestrone sulphatase by a different mechanism. *J. Steroid Biochem. Mol. Biol.* **1996** (in press).
(13) Elger, W.; Schwarz, S.; Hedden, A.; Reddersen, G.; Schneider, B. Sulphamates of various oestrogens - prodrugs with increased systemic and reduced hepatic oestrogenicity at oral application. *J. Steroid Biochem. Mol. Biol.* **1995,** *55*, 395-403.
(14) Li, P. K; Rhodes, M. E.; Jagannathan, S; Johnson, D. A. Memory enhancement mediated by the steroid sulphatase inhibitor oestrone 3-*O*-sulphamate. *J. Endocrinol.* **1995,** *144*, Abstr. P155.
(15) Daynes, R. A.; Araneo, B. A.; Dowell, T. A.; Huang, K.; Dudley, D. Regulation of murine lymphokine production *in vivo.* 3. The lymphoid tissue microenvironment exerts regulatory influences over T-helper cell function. *J*. *Exp*. *Med.* **1990,** *171*, 979-996.
(16) Rook, G. A. W.; Hernandez-Pando, R.; Lightman, S. Hormones, peripherally activated prohormones and regulation of the TH1/TH2 balance. *Immunol. Today* **1994,** *15*, 301-303.

## Claims

1. A sulphamate compound suitable for use as an inhibitor of oestrone sulphatase, wherein the compound is a sulphamate compound having Formula V; 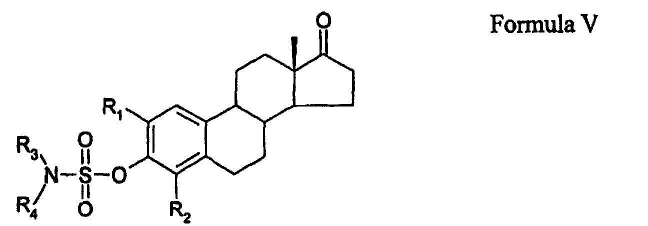 wherein
each of R₁ and R₂ is independently selected from H, alkyl, cycloalkyl, alkoxy, alkenyl, aryl, substituted alkyl, substituted cycloalkyl, substituted alkenyl, substituted aryl, a nitrogen containing group, a S containing group, or a carboxy containing group;
R₁ and R₂ may be the same or different but not both being H; and
each of R₃ and R₄ is independently selected from H, alkyl, cycloalkyl, alkenyl and aryl, wherein at least one of R₃ and R₄ is H.

2. A sulphamate compound suitable for use as an inhibitor of oestrone sulphatase, wherein the compound is a sulphamate compound having Formula II; 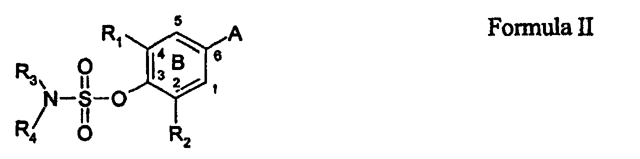 wherein
R₁ is selected from alkyl, cycloalkyl, alkoxy, alkenyl, aryl, substituted alkyl, substituted cycloalkyl, substituted alkenyl, substituted aryl, a nitrogen containing group, a S containing group, or a carboxy containing group;
R₂ is selected from H, alkyl, cycloalkyl, alkoxy, alkenyl, aryl, substituted alkyl, substituted cycloalkyl, substituted alkenyl, substituted aryl, a nitrogen containing group, a S containing group, or a carboxy containing group;
R₁ and R₂ may be the same or different;
each of R₃ and R₄ is independently selected from H, alkyl, cycloalkyl, alkenyl and aryl, wherein at least one of R₃ and R₄ is H;
group A and ring B together are capable of mimicking the A and B rings of oestrone; and
group A is additionally attached to the carbon atom at position 1 of the ring B.

3. Use of a compound in the manufacture of a medicament to inhibit steroid sulphatase activity, wherein the compound is a sulphamate compound having Formula II; 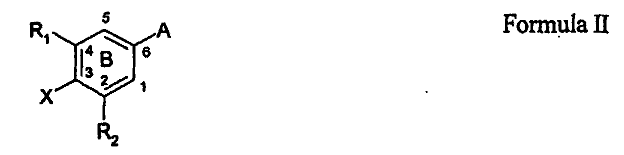 wherein
X is a sulphamate group;
R₁ is selected from alkyl, cycloalkyl, alkoxy, alkenyl, aryl, substituted alkyl, substituted cycloalkyl, substituted alkenyl, substituted aryl, a nitrogen containing group, a S containing group, or a carboxy containing group; and
R₂ is selected from H, alkyl, cycloalkyl, alkoxy, alkenyl, aryl, substituted alkyl, substituted cycloalkyl, substituted alkenyl, substituted aryl, a nitrogen containing group, a S containing group, or a carboxy containing group;
R₁ and R₂ may be the same or different;
wherein group A and ring B together are capable of mimicking the A and B rings of oestrone; and
wherein group A is additionally attached to the carbon atom at position 1 of the ring B.

4. Use of a compound in the manufacture of a medicament to inhibit steroid sulphatase activity, wherein the compound is a sulphamate compound having Formula V; 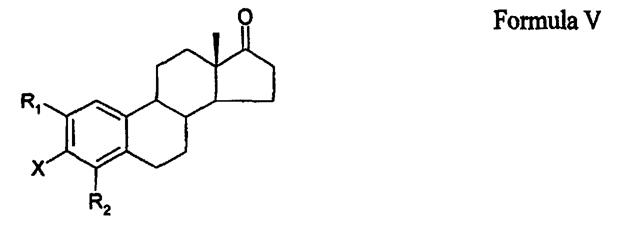 wherein
X is a sulphamate group;
each of R₁ and R₂ is independently selected from H, alkyl, cycloalkyl, alkoxy, alkenyl, aryl, substituted alkyl, substituted cycloalkyl, substituted alkenyl, substituted aryl, a nitrogen containing group, a S containing group, or a carboxy containing group; and
R₁ and R₂ may be the same or different but not both being H.

5. A sulphamate compound according to claim 2 wherein the compound has the Formula V

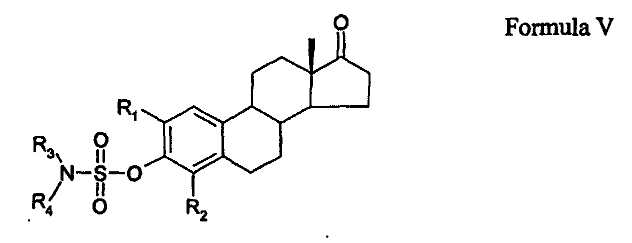

6. A use according to claim 3 wherein the compound has the Formula V

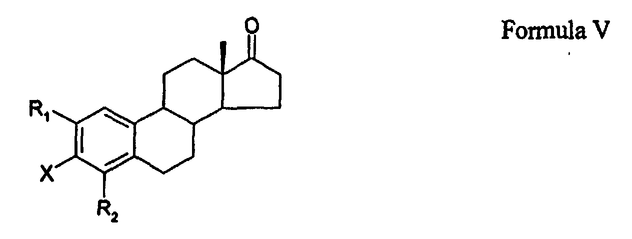

7. A use according to claim 3,4 or 6 wherein the sulphamate group has the Formula III; 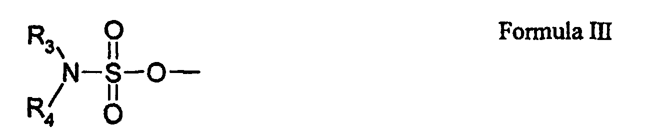 wherein each of R₃ and R₄ is independently selected from H, alkyl, cycloalkyl, alkenyl and aryl, or together represent alkylene optionally containing one or more hetero atoms or groups in the alkylene chain.

8. A sulphamate compound or use according to claim 1, 2 or 7 wherein at least one of R₃ and R₄ is H.

9. A sulphamate compound or use according to claim 8 wherein each of R₃ and R₄ is H.

10. A sulphamate compound or use according to one of claims 2, 3, 5 and 6 wherein
R₁ is selected from C₁₋₆ alkyl, C₁₋₆ cycloalkyl, C₁₋₆ alkenyl, substituted C₁₋₆ alkyl, substituted C₁₋₆ cycloalkyl, substituted C₁₋₆ alkenyl, substituted aryl, a nitrogen containing group, a S containing group, or a carboxy group having from 1-6 carbon atoms; and
R₂ is selected from H, C₁₋₆ alkyl, C₁₋₆ cycloalkyl, C₁₋₆ alkenyl, substituted C₁₋₆ alkyl, substituted C₁₋₆ cycloalkyl, substituted C₁₋₆ alkenyl, substituted aryl, a nitrogen containing group, a S containing group, or a carboxy group having from 1-6 carbon atoms.

11. A sulphamate compound or use according to claim 10 wherein
R₁ is selected from C₁₋₆ alkyl, C₁₋₆ alkenyl, a nitrogen containing group, or a carboxy group having from 1-6 carbon atoms; and
R₂ is selected from H, C₁₋₆ alkyl, C₁₋₆ alkenyl, a nitrogen containing group, or a carboxy group having from 1-6 carbon atoms.

12. A sulphamate compound or use according to claim 11 wherein
R₁ is selected from C₁₋₆ alkyl, C₁₋₆ alkenyl, NO₂, or a carboxy group having from 1-6 carbon atoms; and
R₂ is selected from H, C₁₋₆ alkyl, C₁₋₆ alkenyl, NO₂, or a carboxy group having from 1-6 carbon atoms.

13. A sulphamate compound or use according to claim 12 wherein
R₁ is selected from C₃ alkyl, C₃ alkenyl, NO₂, and H₃CO; and
R₂ is selected from H, C₃ alkyl, C₃ alkenyl, NO₂, and H₃CO.

14. A sulphamate compound or use according to claim 1 or 4 wherein each of R₁ and R₂ is independently selected from H, C₁₋₆ alkyl, C₁₋₆ cycloalkyl, C₁₋₆ alkenyl, substituted C₁₋₆ alkyl, substituted C₁₋₆ cycloalkyl, substituted C₁₋₆ alkenyl, substituted aryl, a nitrogen containing group, a S containing group, or a carboxy group having from 1-6 carbon atoms.

15. A sulphamate compound or use according to claim 14 wherein each of R₁ and R₂ is independently selected from H, C₁₋₆ alkyl, C₁₋₆ alkenyl, a nitrogen containing group, or a carboxy group having from 1-6 carbon atoms.

16. A sulphamate compound or use according to claim 15 wherein each of R₁ and R₂ is independently selected from H, C₁₋₆ alkyl, C₁₋₆ alkenyl, NO₂, or a carboxy group having from 1-6 carbon atoms.

17. A sulphamate compound or use according to claim 16 wherein each of R₁ and R₂ is independently selected from H, C₃ alkyl, C₃ alkenyl, NO₂, or H₃CO.

18. A sulphamate compound or use according to claim 1 wherein the compound is any one of the Formulae VI - IX.

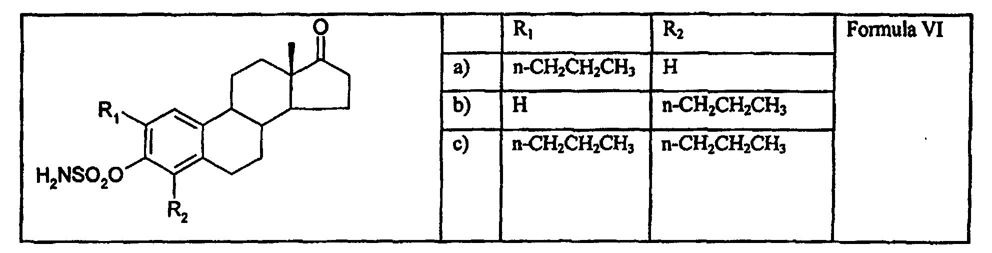

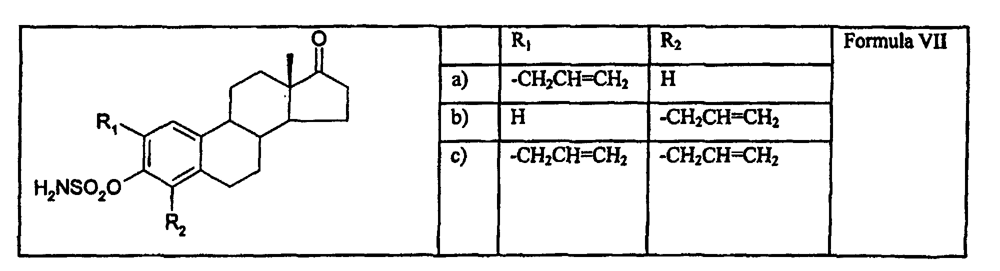

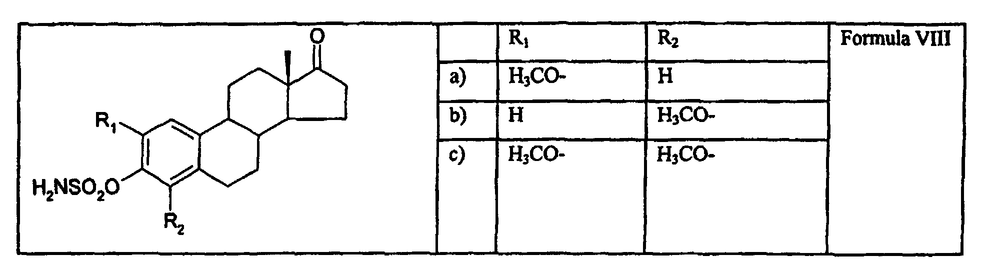

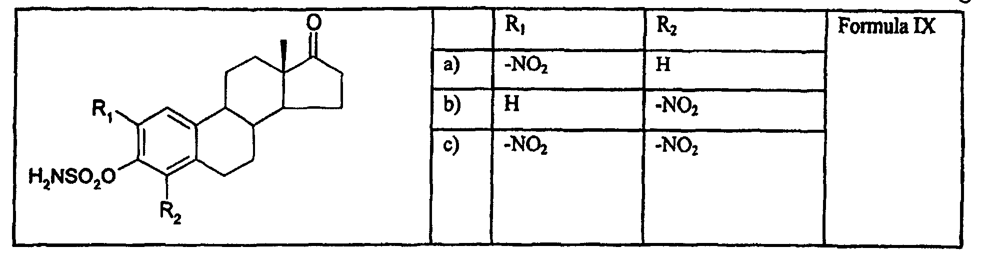

19. A sulphamate compound or use according to any one of the preceding claims wherein the compound is further **characterised by** the feature that if the sulphamate group were to be substituted with a sulphate group to form a sulphate derivative, then the sulphate derivative would be hydrolysable by an enzyme having steroid sulphatase (E.C. 3.1.6.2) activity.

20. A sulphamate compound or use according to any one of claims 1 to 4 wherein R₁ and/or R₂ is an alkoxy group.

21. A sulphamate compound or use according to claim 20 wherein R₁ and/or R₂ is a methoxy group.

22. A sulphamate compound or use according to claim 20 wherein R₁ is an alkoxy group.

23. A sulphamate compound or use according to claim 22 wherein R₁ is a methoxy group.

24. A sulphamate compound or use according to any one of claims 1 to 4 wherein R₁ and/or R₂ is an alkyl group.

25. A sulphamate compound or use according to claim 24 wherein R₁ and/or R₂ is a C₁₋₆ alkyl group.

26. A sulphamate compound or use according to claim 25 wherein R₁ and/or R₂ is an ethyl group.

## Patentansprüche

1. Sulfamatverbindung, geeignet für die Verwendung als ein Inhibitor von Östronsulfatase, wobei die Verbindung eine Sulfamatverbindung ist, welche die Formel V aufweist, 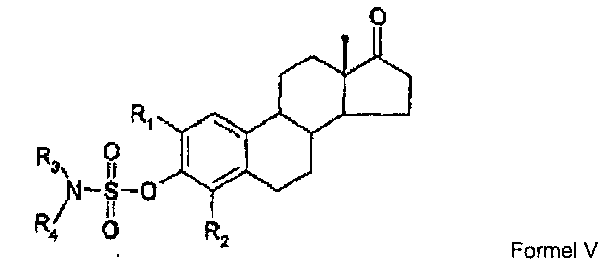 worin
R₁ und R₂ jeweils unabhängig voneinander unter H, Alkyl, Cycloalkyl, Alkoxy, Alkenyl, Aryl, substituiertem Alkyl, substituiertem Cycloalkyl, substituiertem Alkenyl, substituiertem Aryl, einer Stickstoff enthaltenden Gruppe, einer S enthaltenden Gruppe oder einer Carboxy enthaltenden Gruppe ausgewählt sind,
R₁ und R₂ gleich oder verschieden, aber nicht beide H sein können und
R₃ und R₄ jeweils unabhängig voneinander unter H, Alkyl, Cycloalkyl, Alkenyl und Aryl ausgewählt sind, wobei wenigstens eines von R₃ und R₄ H ist.

2. Sulfamatverbindung, geeignet für die Verwendung als ein Inhibitor von Östronsulfatase, wobei die Verbindung eine Sulfamatverbindung ist, welche die Formel II aufweist, 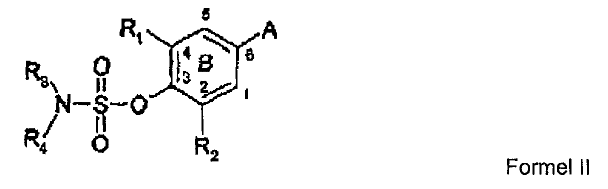 worin
R₁ unter Alkyl, Cycloalkyl, Alkoxy, Alkenyl, Aryl, substituiertem Alkyl, substituiertem Cycloalkyl, substituiertem Alkenyl, substituiertem Aryl, einer Stickstoff enthaltenden Gruppe, einer S enthaltenden Gruppe oder einer Carboxy enthaltenden Gruppe ausgewählt ist,
R₂ unter H, Alkyl, Cycloalkyl, Alkoxy, Alkenyl, Aryl, substituiertem Alkyl, substituiertem Cycloalkyl, substituiertem Alkenyl, substituiertem Aryl, einer Stickstoff enthaltenden Gruppe, einer S enthaltenden Gruppe oder einer Carboxy enthaltenden Gruppe ausgewählt ist,
R₁ und R₂ gleich oder verschieden sein können,
R₃ und R₄ jeweils unabhängig voneinander unter H, Alkyl, Cycloalkyl, Alkenyl und Aryl ausgewählt sind, wobei wenigstens eines von R₃ und R₄ H ist,
die Gruppe A und der Ring B zusammen in der Lage sind, die A- und B-Ringe von Östron nachzuahmen und
die Gruppe A zusätzlich an das Kohlenstoffatom an Position 1 des Rings B gebunden ist.

3. Verwendung einer Verbindung bei der Herstellung eines Medikaments zur Hemmung von Steroidsulfataseaktivität, wobei die Verbindung eine Sulfamatverbindung ist, welche die Formel II aufweist, 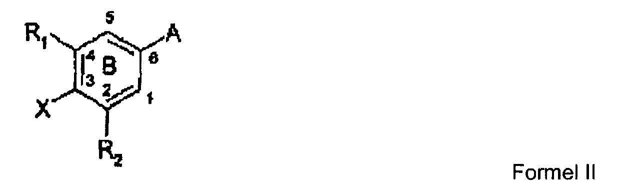 worin
X eine Sulfamatgruppe ist,
R₁ unter Alkyl, Cycloalkyl, Alkoxy, Alkenyl, Aryl, substituiertem Alkyl, substituiertem Cycloalkyl, substituiertem Alkenyl, substituiertem Aryl, einer Stickstoff enthaltenden Gruppe, einer S enthaltenden Gruppe oder einer Carboxy enthaltenden Gruppe ausgewählt ist,
R₂ unter H, Alkyl, Cycloalkyl, Alkoxy, Alkenyl, Aryl, substituiertem Alkyl, substituiertem Cycloalkyl, substituiertem Alkenyl, substituiertem Aryl, einer Stickstoff enthaltenden Gruppe, einer S enthaltenden Gruppe oder einer Carboxy enthaltenden Gruppe ausgewählt ist,
R₁ und R₂ gleich oder verschieden sein können,
die Gruppe A und der Ring B zusammen in der Lage sind, die A- und B-Ringe von Östron nachzuahmen und
die Gruppe A zusätzlich an das Kohlenstoffatom an Position 1 des Rings B gebunden ist.

4. Verwendung einer Verbindung bei der Herstellung eines Medikaments zur Hemmung von Steroidsulfataseaktivität, wobei die Verbindung eine Sulfamatverbindung ist, welche die Formel V aufweist, 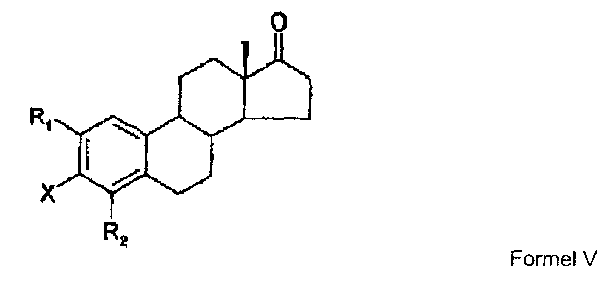 worin
X eine Sulfamatgruppe ist,
R₁ und R₂ jeweils unabhängig voneinander unter H, Alkyl, Cycloalkyl, Alkoxy, Alkenyl, Aryl, substituiertem Alkyl, substituiertem Cycloalkyl, substituiertem Alkenyl, substituiertem Aryl, einer Stickstoff enthaltenden Gruppe, einer S enthaltenden Gruppe oder einer Carboxy enthaltenden Gruppe ausgewählt sind, und
R₁ und R₂ gleich oder verschieden, aber nicht beide H sein können,

5. Sulfamatverbindung nach Anspruch 2, wobei die Verbindung die Formel V aufweist.

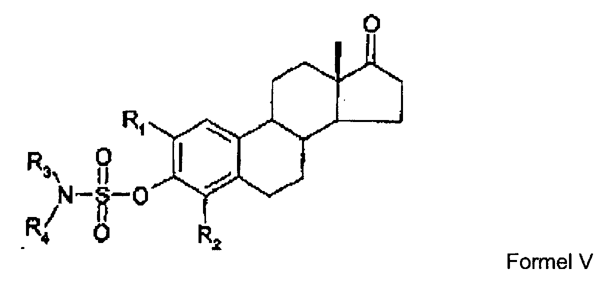

6. Verwendung nach Anspruch 3, wobei die Verbindung die Formel V aufweist.

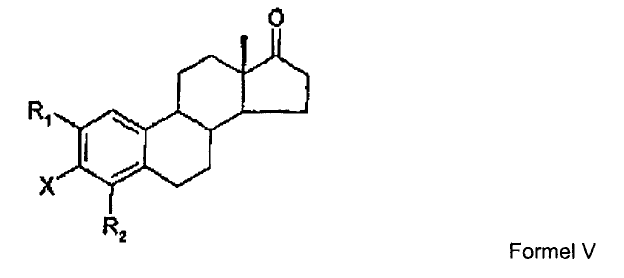

7. Verwendung nach Anspruch 3,4 oder 6, wobei die Sulfamatgruppe die Formel III aufweist, 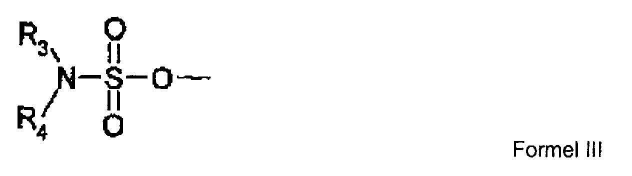 worin R₃ und R₄ jeweils unabhängig voneinander unter H, Alkyl, Cycloalkyl, Alkenyl und Aryl ausgewählt sind oder zusammen Alkylen darstellen, welches wahlweise ein oder mehrere Heteroatome oder Gruppen in der Alkylenkette enthält.

8. Sulfamatverbindung oder Verwendung nach Anspruch 1, 2 oder 7, wobei wenigstens eines von R₃ und R₄ H ist.

9. Sulfamatverbindung oder Verwendung nach Anspruch 8, wobei R₃ und R₄ jeweils H sind.

10. Sulfamatverbindung oder Verwendung nach einem der Ansprüche 2, 3, 5 und 6, wobei
R₁ unter C₁₋₆-Alkyl, C₁₋₆-Cycloalkyl, C₁₋₆-Alkenyl, substituiertem C₁₋₆-Alkyl, substituiertem C₁₋₆-Cycloalkyl, substituiertem C₁₋₆-Alkenyl, substituiertem Aryl, einer Stickstoff enthaltenden Gruppe, einer S enthaltenden Gruppe oder einer Carboxygruppe mit 1-6 Kohlenstoffatomen ausgewählt ist und
R₂ unter H, C₁₋₆-Alkyl, C₁₋₆-Cycloalkyl, C₁₋₆-Alkenyl, substituiertem C₁₋₆-Alkyl, substituiertem C₁₋₆-Cycloalkyl, substituiertem C₁₋₆-Alkenyl, substituiertem Aryl, einer Stickstoff enthaltenden Gruppe, einer S enthaltenden Gruppe oder einer Carboxygruppe mit 1-6 Kohlenstoffatomen ausgewählt ist.

11. Sulfamatverbindung oder Verwendung nach Anspruch 10, wobei
R₁ unter C₁₋₆-Alkyl, C₁₋₆-Alkenyl, einer Stickstoff enthaltenden Gruppe oder einer Carboxygruppe mit 1-6 Kohlenstoffatomen ausgewählt ist und
R₂ unter H, C₁₋₆-Alkyl, C₁₋₆-Alkenyl, einer Stickstoff enthaltenden Gruppe oder einer Carboxygruppe mit 1-6 Kohlenstoffatomen ausgewählt ist.

12. Sulfamatverbindung oder Verwendung nach Anspruch 11, wobei
R₁ unter C₁₋₆-Alkyl, C₁₋₆-Alkenyl, NO₂ oder einer Carboxygruppe mit 1-6 Kohlenstoffatomen ausgewählt ist und
R₂ unter H, C₁₋₆-Alkyl, C₁₋₆-Alkenyl, NO₂ oder einer Carboxygruppe mit 1-6 Kohlenstoffatomen ausgewählt ist.

13. Sulfamatverbindung oder Verwendung nach Anspruch 12, wobei
R₁ unter C₃-Alkyl, C₃-Alkenyl, NO₂ und H₃CO ausgewählt ist und
R₂ unter H, C₃-Alkyl, C₃-Alkenyl, NO₂ und H₃CO ausgewählt ist.

14. Sulfamatverbindung oder Verwendung nach Anspruch 1 oder 4, wobei R₁ und R₂ jeweils unabhängig voneinander unter H, C₁₋₆-Alkyl, C₁₋₆-Cycloalkyl, C₁₋₆-Alkenyl, substituiertem C₁₋₆-Alkyl, substituiertem C₁₋₆-Cycloalkyl, substituiertem C₁₋₆-Alkenyl, substituiertem Aryl, einer Stickstoff enthaltenden Gruppe, einer S enthaltenden Gruppe oder einer Carboxygruppe mit 1-6 Kohlenstoffatomen ausgewählt sind.

15. Sulfamatverbindung oder Verwendung nach Anspruch 14, wobei R₁ und R₂ jeweils unabhängig voneinander unter H, C₁₋₆-Alkyl, C₁₋₆-Alkenyl, einer Stickstoff enthaltenden Gruppe oder einer Carboxygruppe mit 1-6 Kohlenstoffatomen ausgewählt sind.

16. Sulfamatverbindung oder Verwendung nach Anspruch 15, wobei R₁ und R₂ jeweils unabhängig voneinander unter H, C₁₋₆-Alkyl, C₁₋₆-Alkenyl, NO₂ oder einer Carboxygruppe mit 1-6 Kohlenstoffatomen ausgewählt sind.

17. Sulfamatverbindung oder Verwendung nach Anspruch 16, wobei R₁ und R₂ jeweils unabhängig voneinander unter H, C₃-Alkyl, C₃-Alkenyl, NO₂ oder H₃CO ausgewählt sind.

18. Sulfamatverbindung oder Verwendung nach Anspruch 1, wobei die Verbindung eine der Formeln VI-IX aufweist.

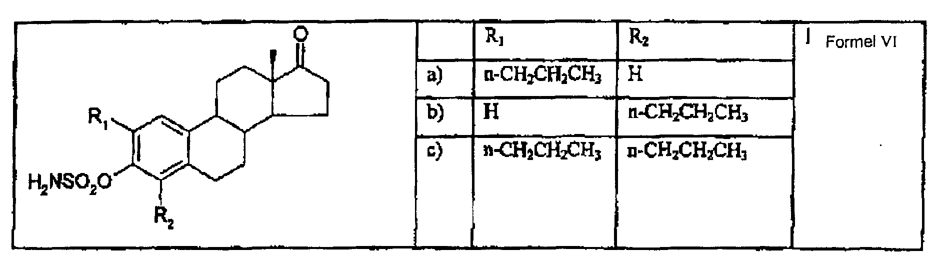

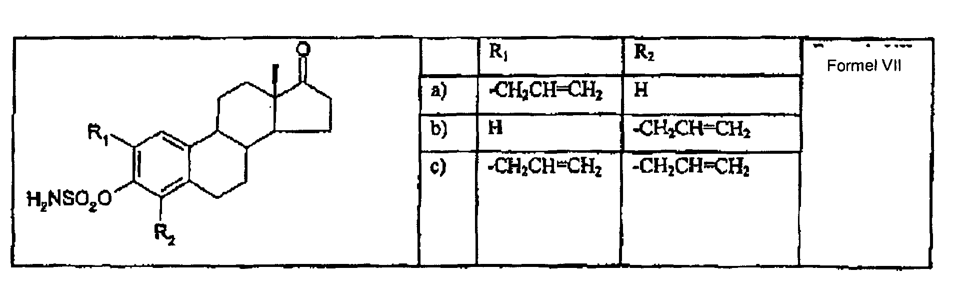

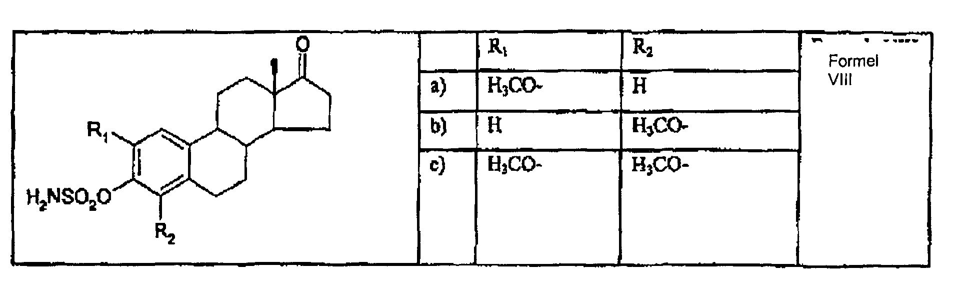

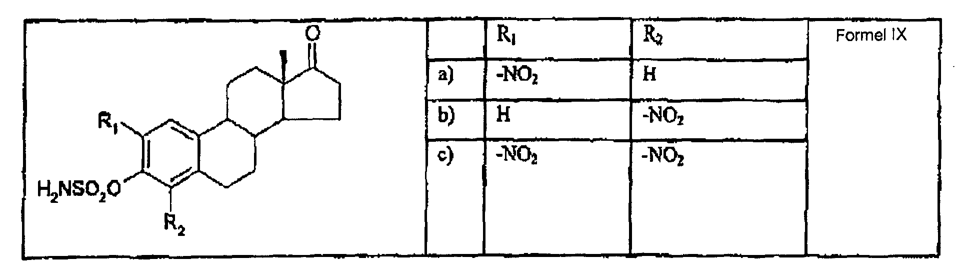

19. Sulfamatverbindung oder Verwendung nach einem der vorangegangenen Ansprüche, wobei die Verbindung weiter durch das Merkmal gekennzeichnet ist, daß, wenn die Sulfamatgruppe durch eine Sulfatgruppe unter Bildung eines Sulfatderivates substituiert wäre, dann das Sulfatderivat durch ein Enzym mit Steroidsulfatase (E.C. 3.1.6.2)-Aktivität hydrolysierbar wäre.

20. Sulfamatverbindung oder Verwendung nach einem der Ansprüche 1 bis 4, wobei R₁ und/oder R₂ eine Alkoxygruppe ist.

21. Sulfamatverbindung oder Verwendung nach Anspruch 20, wobei R₁ und/oder R₂ eine Methoxygruppe ist.

22. Sulfamatverbindung oder Verwendung nach Anspruch 20, wobei R₁ eine Alkoxygruppe ist.

23. Sulfamatverbindung oder Verwendung nach Anspruch 22, wobei R₁ eine Methoxygruppe ist.

24. Sulfamatverbindung oder Verwendung nach einem der Ansprüche 1 bis 4, wobei R₁ und/oder R₂ eine Alkylgruppe ist.

25. Sulfamatverbindung oder Verwendung nach Anspruch 24, wobei R₁ und/oder R₂ eine C₁₋₆-Alkylgruppe ist.

26. Sulfamatverbindung oder Verwendung nach Anspruch 25, wobei R₁ und/oder R₂ eine Ethylgruppe ist.

## Revendications

1. Composé consistant en sulfamate convenable pour l'utilisation comme inhibiteur d'oestrone-sulfatase, ledit composé étant un sulfamate répondant à la formule V ; 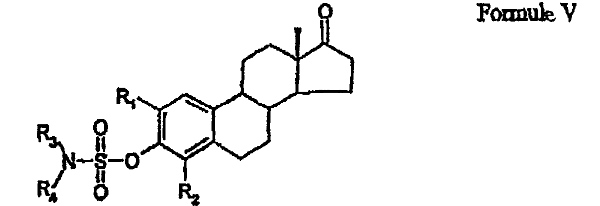 dans laquelle
chacun des groupes R₁ et R₂ est choisi indépendamment entre H, des groupes alkyle, cycloalkyle, alkoxy, alcényle, aryle, alkyle substitué, cycloalkyle substitué, alcényle substitué, aryle substitué, un groupe contenant de l'azote, un groupe contenant S ou un groupe à fonction carboxy ;
R₁ et R₂ peuvent être identiques ou différents mais ne représentent pas l'un et l'autre H ; et
chacun des groupes R₃ et R₄ est choisi indépendamment entre H, des groupes alkyle, cycloalkyle, alcényle et aryle, au moins un des groupes R₃ et R₄ représentant H.

2. Composé consistant en sulfamate convenable pour l'utilisation comme inhibiteur d'oestrone-sulfatase, ledit composé étant un sulfamate répondant à la formule II : 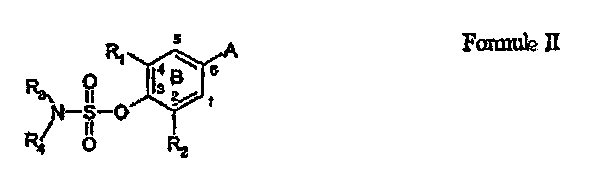 dans laquelle
R₁ est choisi entre des groupes alkyle, cycloalkyle, alkoxy, alcényle, aryle, alkyle substitué, cycloalkyle substitué, alcényle substitué, aryle substitué, un groupe contenant de l'azote, un groupe contenant S ou un groupe à fonction carboxy ;
R₂ est choisi entre H, des groupes alkyle, cycloalkyle, alkoxy, alcényle, aryle, alkyle substitué, cycloalkyle substitué, alcényle substitué, aryle substitué, un groupe contenant de l'azote, un groupe contenant S ou un groupe à fonction carboxy ;
R₁ et R₂ peuvent être identiques ou différents ;
chacun des groupes R₃ et R₄ est choisi indépendamment entre H, des groupes alkyle, cycloalkyle, alcényle et aryle, au moins un des groupes R₃ et R₄ représentant H.
le groupe A et le noyau B, conjointement, sont capables de mimer les noyaux A et B de l'oestrone ; et
le groupe A est fixé en outre à l'atome de carbone en position 1 du noyau B.

3. Utilisation d'un composé dans la production d'un médicament destiné à inhiber l'activité de stéroïde-sulfatase, dans laquelle le composé est un sulfamate répondant à la formule II: 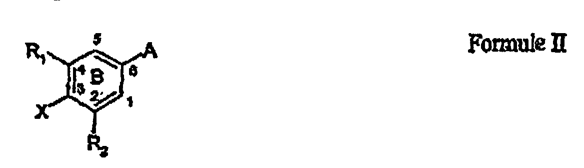 dans laquelle
X représente un groupe sulfamate ;
R₁ est choisi entre des groupes alkyle, cycloalkyle, alkoxy, alcényle, aryle, alkyle substitué, cycloalkyle substitué, alcényle substitué, aryle substitué, un groupe contenant de l'azote, un groupe contenant S ou un groupe à fonction carboxy ; et
R₂ est choisi entre H, des groupes alkyle, cycloalkyle, alkoxy, alcényle, aryle, alkyle substitué, cycloalkyle substitué, alcényle substitué, aryle substitué, un groupe contenant de l'azote, un groupe contenant S ou un groupe à fonction carboxy ;
R₁ et R₂ peuvent être identiques ou différents ;
le groupe A et le noyau B, conjointement, sont capables de mimer les noyaux A et B de l'oestrone ; et
le groupe A est fixé en outre à l'atome de carbone en position 1 du noyau B.

4. Utilisation d'un composé dans la production d'un médicament destiné à inhiber l'activité de stéroïde-sulfatase, dans laquelle le composé est un sulfamate répondant à la formule V : 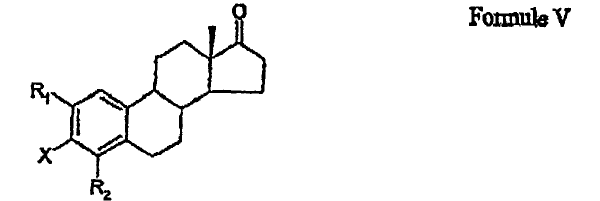 dans laquelle
X représente un groupe sulfamate ;
chacun des groupes R₁ et R₂ est choisi indépendamment entre H, des groupes alkyle, cycloalkyle, alkoxy, alcényle, aryle, alkyle substitué, cycloalkyle substitué, alcényle substitué, aryle substitué, un groupe contenant de l'azote, un groupe contenant S ou un groupe à fonction carboxy ; et
R₁ et R₂ peuvent être identiques ou différents mais ne représentent pas l'un et l'autre H.

5. Composé consistant en sulfamate suivant la revendication 2, qui répond à la formule V

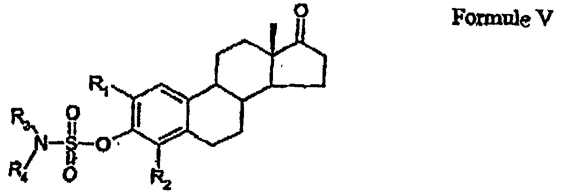

6. Utilisation suivant la revendication 3, dans laquelle le composé répond à la formule V

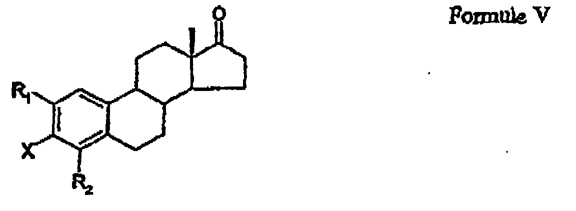

7. Utilisation suivant la revendication 3, 4 ou 6, dans laquelle le groupe sulfamate répond à la formule III 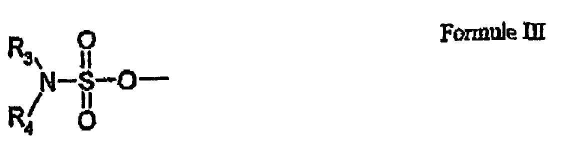 dans laquelle chacun des groupes R3 et R4 est choisi indépendamment entre H, des groupes alkyle, cycloalkyle, alcényle et aryle, ou bien ces groupes, conjointement, représentent un groupe alkylène contenant facultativement un ou plusieurs hétéro-atomes ou groupes hétéro-atomiques dans la chaîne alkylène.

8. Composé consistant en sulfamate ou utilisation suivant la revendication 1, 2 ou 7, dans lequel au moins un des groupes R₃ et R₄ représente H.

9. Composé consistant en sulfamate ou utilisation suivant la revendication 8, dans lequel chacun des groupes R₃ et R₄ représente H.

10. Composé consistant en sulfamate ou utilisation suivant une des revendications 2, 3, 5 et 6, dans lequel
R₁ est choisi entre des groupes alkyle en C₁ à C₆, cycloalkyle en C₁ à C₆, alcényle en C₁ à C₆, alkyle en C₁ à C₆ substitué, cycloalkyle en C₁ à C₆ substitué, alcényle en C₁ à C₆ substitué, aryle substitué, un groupe contenant de l'azote, un groupe contenant S ou un groupe carboxy ayant 1 à 6 atomes de carbone ; et
R₂ est choisi entre H, des groupes alkyle en C₁ à C₆, cycloalkyle en C₁ à C₆, alcényle en C₁ à C₆, alkyle en C₁ à C₆ substitué, cycloalkyle en C₁ à C₆ substitué, alcényle en C₁ à C₆ substitué, aryle substitué, un groupe contenant de l'azote, un groupe contenant S et un groupe carboxy ayant 1 à 6 atomes de carbone.

11. Composé consistant en sulfamate ou utilisation suivant la revendication 10, dans lequel
R₁ est choisi entre des groupes alkyle en C₁ à C₆, alcényle en C₁ à C₆, un groupe contenant de l'azote et un groupe carboxy ayant 1 à 6 atomes de carbone ; et
R₂ est choisi entre H, des groupes alkyle en C₁ à C₆, alcényle en C₁ à C₆, un groupe contenant de l'azote ou un groupe carboxy ayant 1 à 6 atomes de carbone.

12. Composé consistant en sulfamate ou utilisation suivant la revendication 11, dans lequel
R₁ est choisi entre des groupes alkyle en C₁ à C₆, alcényle en C₁ à C₆, NO₂, ou un groupe carboxy ayant 1 à 6 atomes de carbone ; et
R₂ est choisi entre H, des groupes alkyle en C₁ à C₆, alcényle en C₁ à C₆, NO₂, ou un groupe carboxy ayant 1 à 6 atomes de carbone.

13. Composé consistant en sulfamate ou utilisation suivant la revendication 12, dans lequel
R₁ est choisi entre des groupes alkyle en C₃, alcényle, NO₂ et H₃CO ; et
R₂ est choisi entre H, des groupes alkyle en C₃, alcényle en C₃, NO₂ et H₃CO.

14. Composé consistant en sulfamate ou utilisation suivant la revendication 1 ou 4, dans lequel chacun des groupes R₁ et R₂ est choisi indépendamment entre H, des groupes alkyle en C₁ à C₆, cycloalkyle en C₁ à C₆, alcényle en C₁ à C₆, alkyle en C₁ à C₆ substitué, cycloalkyle en C₁ à C₆ substitué, alcényle en C₁ à C₆ substitué, aryle substitué, un groupe contenant de l'azote, un groupe contenant S ou un groupe carboxy ayant 1 à 6 atomes de carbone.

15. Composé consistant en sulfamate ou utilisation suivant la revendication 14, dans lequel chacun des groupes R₁ et R₂ est choisi indépendamment entre H, des groupes alkyle en C₁ à C₆, alcényle en C₁ à C₆, un groupe contenant de l'azote ou un groupe carboxy ayant 1 à 6 atomes de carbone.

16. Composé consistant en sulfamate ou utilisation suivant la revendication 15, dans lequel chacun des groupes R₁ et R₂ est choisi indépendamment entre H, des groupes alkyle en C₁ à C₆, alcényle en C₁ à C₆, NO₂, ou un groupe carboxy ayant 1 à 6 atomes de carbone.

17. Composé consistant en sulfamate ou utilisation suivant la revendication 16, dans lequel chacun des groupes R₁ et R₂ est choisi indépendamment entre H, des groupes alkyle en C₃, alcényle en C₃, NO₂ et H₃CO.

18. Composé consistant en sulfamate ou utilisation suivant la revendication 1, qui est l'un quelconque des composés de formules VI à IX

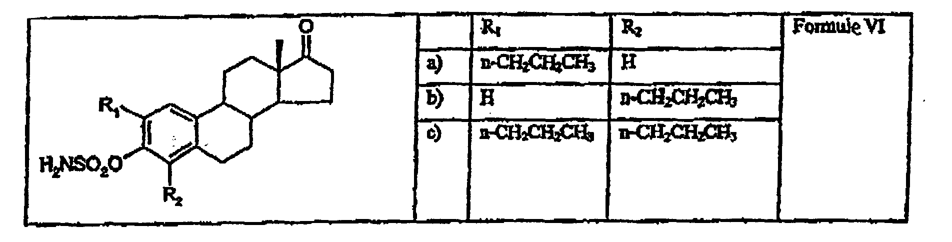

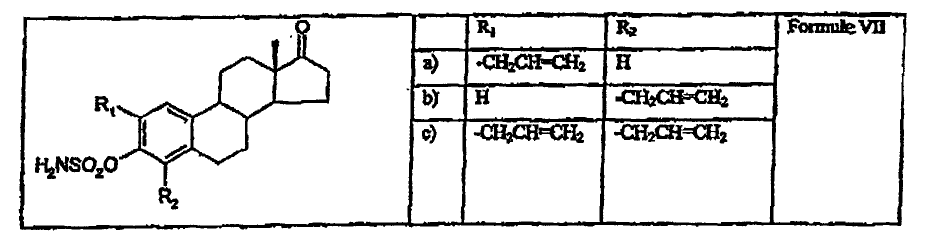

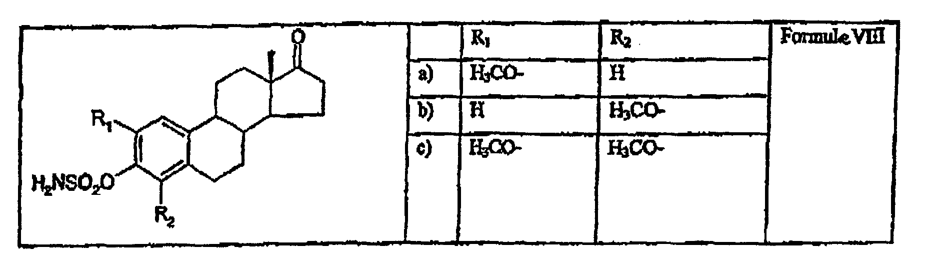

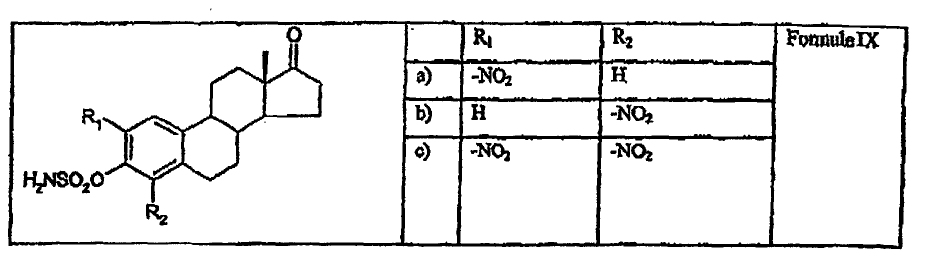

19. Composé consistant en sulfamate ou utilisation suivant l'une quelconque des revendications précédentes, dans lequel le composé est **caractérisé en outre par le fait que**, si le groupe sulfamate devait être substitué par un groupe sulphate pour former un dérivé consistant en sulphate, alors le dérivé consistant en sulphate serait hydrolysable par une enzyme ayant une activité de stéroïde-sulfatase (E.C. 3.1.6.2).

20. Composé consistant en sulfamate ou utilisation suivant l'une quelconque des revendications 1 à 4, dans lequel R₁ et/ou R₂ représente un groupe alkoxy.

21. Composé consistant en sulfamate ou utilisation suivant la revendication 20, dans lequel R₁ et/ou R₂ représente un groupe méthoxy.

22. Composé consistant en sulfamate ou utilisation suivant la revendication 20, dans lequel R₁ représente un groupe alkoxy.

23. Composé consistant en sulfamate ou utilisation suivant la revendication 22, dans lequel R₁ représente un groupe méthoxy.

24. Composé consistant en sulfamate ou utilisation suivant l'une quelconque des revendications 1 à 4, dans lequel R₁ et/ou R₂ représente un groupe alkyle.

25. Composé consistant en sulfamate ou utilisation suivant la revendication 24, dans lequel R₁ et/ou R₂ représente un groupe alkyle en C₁ à C₆.

26. Composé consistant en sulfamate ou utilisation suivant la revendication 25, dans lequel R₁ et/ou R₂ représente un groupe éthyle.
